# EUROPEAN PATENT APPLICATION

(11) **EP 2 143 442 A1**
(43) Date of publication of application: **13.01.2010**
(21) Application number: 08012637.8
(22) Date of filing: 11.07.2008
(51) Int. Cl.: A61K 41/00, A61K 47/48, A61P 35/00, C07K 14/81, A61K 38/57

(54) **Treatment of solid tumors with tissue inhibitors of metalloproteinases(TIMPs)**

(71) Applicant: Nelson, Peter Jon, 80336 München (DE)
(72) Inventor: Nelson, Peter Jon, 80336 München (DE)
(74) Representative: Grund, Martin

(57) **Abstract**

The invention belongs to the field of therapies for solid cancers, in particular, skin cancer. Compositions comprising tissue inhibitors of metalloproteinases (TIMPs) linked to anchors for anchoring TIMP into a cell membrane and heatlabile vesicles are provided. Also provided are uses of TIMP or TIMP constructs for treating solid cancers in combination with hyperthermic treatment.

## Description

### FIELD OF THE INVENTION

The invention belongs to the field of therapies for solid cancers, in particular, skin cancer. Compositions comprising tissue inhibitors of metalloproteinases (TIMPs) linked to anchors for anchoring TIMP into a cell membrane and heatlabile vesicles are provided. Also provided are uses of TIMP or TIMP constructs for treating solid cancers in combination with hyperthermic treatment.

### BACKGROUND OF THE INVENTION

The combined application of hyperthermia with chemotherapy and/or radiation in treatment of solid cancers is known (1, 2). Solid cancers appear in many forms, for example, breast cancer, prostate cancer, sarcomas, and skin cancer. One form of skin cancer is melanoma.

Melanoma is the most aggressive form of skin cancer and is notoriously resistant to current modalities of cancer therapy (3). A large set of genetic, functional and biochemical studies suggest that melanoma cells become resistant to chemotherapeutic drugs by exploiting their intrinsic resistance to apoptosis and by reprogramming their proliferation and survival pathways during melanoma progression (4, 5). Hyperthermic treatment has been shown to effect the antigenicity of human melanoma cells, their recognition by cytotoxic lymphocytes and immune cell induced apoptosis (6).

The identification of molecules involved in the regulation and execution of immune mediated apoptosis have provided insights into the molecular basis for melanoma chemoresistance (4, 7). An important challenge today is the application of therapeutic strategies that are potent enough to compensate, bypass or modulate the cell death defects associated with melanoma to improve the prognosis of patients at late stages of the disease (7).

The matrix metalloproteinases (MMPs) are a family of zinc-dependent endopeptidases that collectively degrade components of the extracellular matrix. MMPs have been implicated in tissue remodelling, tumor invasion, resistance to apoptosis and metastasis (8, 9). MMP activity is regulated at many levels including by association with the four endogenous MMP inhibitors, the tissue inhibitor of matrix metalloproteinases (TIMP)-1, -2, -3 and -4 (10). In vivo, the balance between MMP and TIMP activities determines whether matrix resorption or deposition occurs (11). While TIMPs inhibit MMP activity and thus can inhibit tumor growth and metastasis, TIMPs are also multifunctional proteins that have been shown to also regulate cell proliferation, apoptosis and angiogenesis.

TIMP-1 is a broadly acting MMP inhibitor (10). It is a soluble protein that can sometimes be detected on the cell surface through its association with surface bound proteins (12, 13). The overall role of TIMP-1 in cancer biology remains the subject of conflicting reports (14, 15). TIMP-1 has been linked to angiogenesis, cell migration, proliferation, tumor growth, metastases and tumor prognosis (16) (15).

Proteins anchored by glycosylphosphatidylinositol (GPI) when purified and added to cells in vitro, are efficiently incorporated into their surface membranes (17-19). This protein engineering of cell surfaces using GPI-anchors has been used to study protein function (17-19). We recently reported the generation of a fusion protein based on human TIMP-1 combined with a GPI anchor (derived from LFA-3) to generate a recombinant reagent that could be used to focus exogenously added TIMP-1 onto cell surfaces independently of protein-protein interactions (18-20). TIMP-1-GPI treatment was found to slow cancer cell growth in vitro and to increase the sensitivity of renal cell carcinoma cells (RCC) to FAS induced apoptosis (19). Initial results demonstrated that, unlike RCC, melanoma remained largely resistant to TIMP-1-GPI treatment. The problem to be solved was the provision of new composition, menthods, and uses for the treatment of skin cancer. Therefore, we sought to explore the effects of exogenously added TIMP-1-GPI protein in combination with thermal shock on the biology of melanoma cells in vitro. Hyperthermic treatment was found to synergize with TIMP-1-GPI to render melanoma cells sensitive to immune mediated apoptosis. The use of thermolabile liposomes in the context of hyperthermic treatment is ideally suited for the targeted application of TIMP-1-GPI agent in tumor therapy.

### SUMMARY OF THE INVENTION

The invention relates to the use of a construct, wherein said construct comprises a tissue matrix metalloproteinase (TIMP) linked to an anchor for anchoring TIMP into a cell membrane for the treatment of a solid tumor. Particularly preferred is the use wherein the tumor is skin cancer, osteosarcoma or breast cancer. Another embodiment relates to the use wherein the skin cancer is a melanoma.

In a further embodiment, the anchor is glycosylphosphatidylinositol (GPI).

In a further embodiment, TIMP is selected from the group consisting of TIMP-1, TIMP-2, TIMP-3, or TIMP-4.

In a further embodiment TIMP is to be administered in vesicles. In a further embodiment, the vesicles and/or liposomes are heatlabile.

In a further embodiment, the constructs are to be administered together with hyperthermic treatment. In a further embodiment, the hyperthermic treatment is to be performed with a sub lethal thermal dose. In a further embodiment, the sub lethal thermal dose is to be administered at a temperature of between 40 and 43 degree centigrade. In a further embodiment, said temperature is between 41 and 42 degree centigrade. In a further embodiment, the temperature is 41.8 degree centrigade. In a further embodiment, the thermal dose is to be administered for 0.5 to 10 hours. In a further embodiment, the thermal dose is to be administered for 0.5 to 8 hours. In a further embodiment, the thermal dose is to be administered for 1 to 6 hours. In a further embodiment, the thermal dose is to be administered for 1 to 4 hours. In a further embodiment, the thermal dose is to be administered for 2 hours.

Another embodiment relates to a composition comprising TIMP linked to an anchor for anchoring the TIMP into a cell membrane and a heat labile vesicle.

Another embodiment relates to a kit comprising TIMP linked to an anchor for anchoring the TIMP into a cell membrane and a heating device. In a further embodiment, the kit is further comprising a heat labile vesicle.

### BRIEF DESCRIPTION OF FIGURES

Figure 1
   TIMP-1-GPI incorporation into cell membranes of melanoma cells.
   To demonstrate incorporation of GPI-TIMP-1 protein into cell membranes, purified TIMP-1-GPI or control rhTIMP-1 was added to native SK-MEL23 and WM 226-4 cells. TIMP-1 was detected on the cell surface by FACS analysis. Grey histograms are the isotype control staining, solid-line histograms represent TIMP-1 antibody staining.
Figure 2
   Clonogenic assay was used to assess the survival and proliferation of hyperthermic/TIMP-1-GPI treated melanoma cells
   Survival of melanoma cells after heat and TIMP-1-GPI treatment (a) SK-MEL23 and (b) WM 226-4 at 37°C and 41.8°C for 2 h as described in Materials and Methods. The surviving clones from two independent flasks were counted per experimental condition. The mean values with standard deviation are shown. The effect of increasing levels of TIMP-1-GPI or rhTIMP-1 control protein on the proliferation of (c) SK-MEL23 and (d) WM 226-4 was measured using MTT assay (6). MTT was added after 24 h. While TIMP-1-GPI alone inhibited proliferation of the melanoma cells, hyperthermic treatment amplified the effect. rhTIMP-1-GPI did not effect proliferation of the melanoma cells. For each experiment at least six wells were analysed per experimental condition and time point. The mean values with standard deviation are shown.
Figure 3
   TIMP-1-GPI inhibits the release of proMMP-2 and proMMP-9 from melanoma cells.
   Zymography was used to study the secretion of MMP-2 and MMP-9 from WM-226-4 and SK-MEL23. The cells were treated with different concentrations of TIMP-1-GPI, or control rhTIMP-1, and after 48 h the serum-free culture supernatant was removed and analyzed by gelatinase zymography.
Figure 4
   TIMP-1-GPI in combination with heat shock renders cells sensitive to FAS-induced apoptosis in melanoma cells.
   FAS expression on the melanomoa cell lines SK-MEL23, WM-226, WM115, 624.38 MEL and 93.04A12MEL were assessed by flow cytometry using a non-activating anti-FAS mAB (L-958) (a). Untreated cells and cells treated with 14 ng/ml TIMP-1-GPI or 14 ng/ml rhTIMP-1 control protein for 72 h were then stained with L-958 and analyzed.
   The binding of annexin V-fluoroisothiocyanate (FITC) and incorporation of propidium iodide (PI) into the melanoma cell lines after treatment with L-957 for an additional 24 h was used to detect induced FAS mediated apoptosis by flow cytometry for (b) SK-MEL23, (c) WM266, (d) MEL 624.38, (e) WM115 and (f) 93.04A12MEL.
Figure 5
   TIMP-1-GPI and hyperthermic treatment of melanoma cells moderates expression of proteins linked to apoptosis
   The effect of TIMP-1-GPI treatment on the expression of Hsp70, Bcl-2, Bcl-XL, Bax and Apaf-1 was determined by Western blot and protein specific antibody reagents. Following a 72 h preincubation period with 14 ng/ml of TIMP-1-GPI or 14 ng/ml rhTIMP-1 control protein with and without hyperthermic treatment, the melanoma cells were stimulated with 1 µg/ml L-957 (or control isotype antibody) for an additional 24 h. The level of Hsp70, Bcl-2, Bcl-XL, Bax and Apaf-1 protein was then determined in (a) SK-MEL23 and (b) WM226-4.
Figure 6
   Downregulation of TGF-β and induction of IL-10 by a combination of TIMP-1-GPI and hyperthermic treatment, melanoma cells
   Specific ELISA was used to compare total TGF-β protein to that of active TGF-β̃. The active form of TGF-β was effectively reduced by treatment with TIMP-1-GPI and hyperthermia. (a) SK-MEL23 and (b) WM226-4. IL-10 specific ELISA assay was used to measure the release of IL-10 from the melanoma cells before or after single or combined TIMP-1-GPI/hyperthermic treatment in (c) SK-MEL 23 and (d) WM226-4.

### DETAILED DESCRIPTION

### DEFINITIONS

With the term "TIMP" as used herein is meant an endogenous tissue inhibitor of metalloproteinases, which is known to be involved in physiological/biological functions including the inhibition of active matrix metalloproteinases, regulation of pro MMP activation, cell growth, and the modulation of angiogenesis. The human "TIMP family" contains four members, TIMP-1, TIMP-2, TIMP-3, and TIMP-4. One preferred member used in the present invention, TIMP-I, is a secreted protein that can be detected on the cell surface through its interaction with surface proteins (Bode & Maskos, 2003).

The term "fusion construct" or "TIMP fusion construct" as used herein refer to both the nucleic acid molecule and the amino acid molecule encoded thereby.

The invention specifically relates to nucleic acids containing a nucleotide sequence including the sequence defined by SEQ ID NOS: 1-5, or a homolog thereof, or unique fragments thereof as set forth in WO2007039109 (herewith incorporated by reference) and also set forth in the attached sequence listing.

In the present invention, the sequence of a nucleic acid molecule that encodes the resulting protein is considered homologous to a second nucleic acid molecule if the nucleotide sequence of the first nucleic acid molecule is at least about 70% homologous, preferably at least about 80% homologous, and more preferably at least about 90% homologous to the sequence of the second nucleic acid molecule. Homology between two nucleic acid sequences may be readily determined using the known BLASTN algorithm (Altschul, et al. ,1990) with default settings. As a further example, another known test for ascertaining the homology of two nucleic acid sequences is whether they hybridize under normal hybridization conditions, preferably under stringent hybridization conditions.

Given the nucleic acid sequence disclosed herein, the skilled person can readily design nucleic acid structures having particular functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as in situ hybridization, can be constructed. Numerous methods for labeling such probes with radioisotopes, fluorescent tags, enzymes, and binding moieties (e.g., biotin) are known, thus the probes of the invention can be readily adapted for easy detectability.

The protein encoded by the nucleic acid of the present invention further includes functional homologs. A protein is considered a functional homolog of another protein for a specific function, as described below, if the homolog has the same function as the other protein. The homolog can be, for example, a fragment of the protein, or a substitution, addition, or deletion mutant of the protein.

Determination of whether two amino acid sequences are substantially homologous is, for the purpose of the present invention, based on FASTA searches according to Pearson & Lipman (1988). For example, the amino acid sequence of a first protein is considered homologous to that of a second protein if the amino acid sequence of the first protein has at least about 70% amino acid sequence identity, preferably at least about 80% identity, and more preferably at least about 95% identity, with the sequence of the second protein.

The possibility of substituting one amino acid in a sequence with an equivalent amino acid is well-known. Groups of amino acids known to be equivalent include:
(a) AIa(A), Ser(S), Thr(T), Pro(P), GIy(G); (b) Asn(N), Asp(D), GIu(E), GIn(Q);
(c) His(H), Arg(R), Lys(K);
(d) Met(M), Leu(L), He(I), VaI(V); and (e) Phe(F), Tyr(Y), Trp(W).

Substitutions, additions, and/or deletions in the amino acid sequences can be made as long as the protein encoded by the nucleic acid of the invention continues to satisfy the functional criteria described herein. An amino acid sequence that is substantially the same as another sequence, but that differs from the other sequence by means of one or more substitutions, additions, and/or deletions, is considered to be an equivalent sequence.

Preferably, less than 20%, more preferably less than 10%, and still more preferably less than 5%, of the number of amino acid residues in a sequence are substituted for, added to, or deleted from the protein encoded by the nucleic acid of the invention.

With the term "MMP" as used herein is meant a matrix metalloproteinase that belongs to the MMP superfamily as represented by at least 26 extracellular matrix-degrading metalloendopeptidases that are acting during tissue development and differentiation, cellular infiltration, wound healing, and as moderators of the immune response.

With the term "GPI" as used herein is meant glycoinositol phospholipids, in particular, glycosylphosphatidlyinositol as described in Medof et al, 1996. These phospholipid-like anchors have a common structure for membrane attachment irrespective of protein function. GPI anchoring units are composed of a linear glycan containing a phosphoethanolamine, three mannose residues, and a non-acetylated glucosamine linked to an inositol phospholipid. The GPI sequence contains the signals that direct GPI anchoring.

A "TIMP-GPI" fusion construct as used herein relates to TIMP that is fused directly to a GPI-linkage sequence. The TIMP-GPI fusion construct is designed by substituting the 3'-mRNA or cDNA end sequence of naturally GPI-anchored proteins (i.e., a sequence that contains the signals that direct GPI anchoring) for the endogenous TIMP 3'-mRNA or cDNA end sequence.

With the term "FAS" is meant a member of the tumor necrosis factor/nerve growth factor receptor family that induces apoptosis independent of TNF-[alpha]. Other abbreviations known in the art for FAS are Apol (= Apoptosis inducing protein 1) and CD95.

The term "regeneration" generally refers to restoring the integrity of traumatized or otherwise injured tissue. This term can include the processes of wound healing, tissue repair, and other types of restorative activities occurring at the location where a physiological insult and ensuing tissue damage has occurred. Use of tissue matrix metalloproteinase (TIMP) linked to an anchor for anchoring the TIMP into a cell membrane for the treatment of malignant tumors.

The term "vesicle" in the sense of the invention is meant to relate to a structure that can contain TIMP and prevents TIMP from interacting with any components with exemption of the vesicle. In particular, a vesicle has sphere-like appearance and consists of an outer hydrophobic layer encapsulating a hydrophilic interior. The hydrophobic layer can originate from any material that has the capacity to form vesicles. Such material preferentially consists of amphiphatic molecules, i.e., molecules having a hydrophilic and a hydrophobic part. If amphiphatic molecules assemble into a single bilayer encapsulating a hydrophobic interior a unilamminar vesicle is formed. However, multilamminar vesicles, i.e. vesicles having multiple bilayers are also contemplated. Moreover, the use of micelles, i.e., entities of amphiphatic molecules that are no vesicles because they do not contain any hydrophilic content with the exception of TIMP is also contemplated. Micelles consist of aggregates of amphiphatic molecules that form in hydrophilic (micelles) or hydrophobic (inverse micelles) environment. The inclusion of other molecules into the vesicles that are not amphiphatic is also envisioned.

The term "liposome" refers to a certain type of vesicle wherein the amphiphatic molecules are derived from naturally occurring or artificial derivatives of lipids. Examples for such amphiphatic derivatives of lipids are phospholipids, cholesterol, and others.

The term "heatlabile vesicle" or "heatlabile liposome" refers to certain types of vesicles and/or liposomes that contain molecules either amphiphatic or not that render the vesicle or liposome heatlabile,i.e. these entities are stable only within a certain temperature range but disassemble in another temperature range. If vesicles/liposomes diassemble they give up their shape and/or release their content. The preferred released content are the TIMP constructs of the invention. Preferentially, those heatlabile vesicles and/liposomes are stable at 37°C and unstable at temperatures above that value. Such vesicles are disclosed in USPTO Application No: 20060165767; C. Wichmanna "Liposomes for microcompartmentation of enzymes and their influence on catalytic activity", Biochemical and Biophysical Research Communications, Volume 310, Issue 4, 31 October 2003, Pages 1104-1110; S Zellmer, G Cevc Journal of drug targeting (J Drug Target) Vol. 4 Issue 1 Pg. 19-29 (1996) ISSN: 1061-186X SWITZERLAND, all incorporated in their entirety by reference. An especially preferred heatlabile liposome was described by Lindner and colleagues ("Novel temperature-sensitive liposomes with prolonged circulation time", Clin. Cancer Res. 2004 Mar 15;10(6):2168-78, incorporated in its entirety by reference). Heatlabile liposomes comprising 1.2-dipalmitoyl-sn-glycero-3-phosphoglyceroglycerol (DPPGOG), and/or 1.2-dipalmitoyl-sn-glycero-3-phosphocholine and/or 1.2-distearoyl-sn-glycero-3-phosphocholine are especially preferred for mildly hyperthermic conditions (41-42 degrees C).

The term "solid tumor" relates an abnormal mass of tissue that usually does not contain cysts or liquid areas. Solid tumors may be benign (not cancerous), or malignant (cancerous). Different types of solid tumors are named for the type of cells that form them. Examples of solid tumors are sarcomas, carcinomas, and lymphomas. Leukemias (cancers of the blood) generally do not form solid tumors.

Melanoma is a highly malignant tumor of the skin that is characterized by a resistance to common therapeutic modalities (4, 6, 7). The relatively weak responsiveness of melanoma to chemotherapy and immunotherapy is based in part on a general resistance of the tumor to apoptosis. We have previously shown that the engineering of cells by exogenous addition of GPI-TIMP-1 can elicit enhanced as well as novel TIMP-1 biologic activities (18, 19). Exogenously administered TIMP-1-GPI efficiently inserted into the cell membranes of melanoma cells and in combination with sub-lethal thermal dose induced a variety of therapeutically relevant effects.

TIMP-1-GPI treatment alone lead to a dose-dependent reduction in the clonogenic survival capacity of the melanoma cells. This effect was augmented by thermal stress. Hyperthermia as well as treatment with TIMP-1-GPI increased the expression of FAS receptor in all the melanoma cell lines tested. The strongest effect was again achieved by a combination of hyperthermia and TIMP-1-GPI-treatment. Neither TIMP-1-GPI, nor sub-lethal levels of heat shock alone, efficiently increased sensitivity of the melanoma cell lines tested to FAS-mediated apoptosis. However, co-treatment with TIMP-1-GPI and hyperthermia rendered all cell lines tested sensitive to FAS-induced apoptosis.

The FAS-apoptosis pathway is regulated by caspase activation (30, 36). Mitochondria are crucial regulators of apoptosis through several pathways and their function in apoptosis is tightly controlled by the actions of a set of regulatory proteins (30, 37). The upstream events leading to caspase activation are controlled by the overall balance between pro- and anti-apoptotic BCL-2-family proteins (37). The general inducibility of apoptosis in the treated melanoma cells was contrasted with the level of expression of pro- and anti-apoptotic BCL-2 proteins. The pro-apoptotic multidomain protein Bax and the anti-apoptotic Bcl-2 and Bcl-XL proteins showed moderate basal expression in each of the melanoma cell lines analyzed. GPI-TIMP-1 treatment in combination of heat shock resulted in reduced expression of Bcl-2 and Bcl-XL proteins and a corresponding increase in the Bax protein. This shift towards a higher concentration of pro-apoptotic proteins may be one reason for the increased sensitivity of the TIMP-1 surface engineered melanoma cells to FAS-mediated apoptosis. This effect was found to be augmented by sub-lethal hyperthermic treatment.

Apoptosis protease-activating factor-1 (Apaf-1) is also a key regulator of the mitochondrial apoptotic pathway, being the central element of the multimeric apoptosome formed by procaspase 9, cytochrome c, and Apaf-1 itself (38). The role of Apaf-1 is generally described as pro apoptotic. A reduced expression of Apaf-1 is thought to compromise the apoptotic response in melanoma cells (7). Apaf-1 expression has been shown to inversely correlate with the chemosensitivity of melanoma and Apaf-1-negative melanomas are unable to execute a typical apoptotic program in response to p53 activation and are chemoresistant (7). In both melanoma cell lines tested, treatment of the lines with TIMP-1-GPI in combination with thermal dose resulted in an increase in Apaf-1 expression. Treatment with TIMP-1-GPI in combination of heat shock alters the balance of a series of apoptosis regulating proteins to elicit a more "pro-apoptotic" expression profile. This effect appeared to parallel a dose-dependent suppression of proliferation of the melanoma cells.

Growth regulatory proteins of the transforming growth factor-beta family (TGF-β) are one of the few classes of endogenous inhibitors of cell growth (39). Generally, an increase in the expression of TGF-β is associated with human cancers (in vivo) (40). TGF-β has been shown to have a regulatory effect on melanoma invasion and also on the anti-tumor immune response (40). TGF-β has been shown to suppress the development of effector activity in T cells through an inhibition of the expression of cytotoxins in cytotoxic T cells (35, 39, 41). TGF-β can also suppress the expression of MHC class I and class II genes. While this should render the cells more susceptible to recognition by natural killer (NK) cells, TGF-β has been shown to inhibit NK activation (39). TGF-β is produced as a latent inactive form, which is cleaved to gain activity. The cleavage of TGF-β from latent to active forms is mediated at least in part by MMPs (33, 34). The treatment of cells with the TIMP-1-GPI or hyperthermic stress alone did not appear to significantly influence cleavage to the mature protein, however, the combined effect of TIMP-1-GPI with thermal stress lead to a pronounced reduction in the generation of the active form of the protein.

The combination of heat treatment with TIMP-1-GPI also led to a strong increase in the secretion of IL-10. The role of IL-10 in the immunobiology of melanoma is controversial (42, 43). While some reports have suggested that IL-10 expression is found at higher levels in melanoma metastasis, other reports documented higher levels in regressing lesions suggesting an anti tumor role for this cytokine (35, 42). IL-10 is thought to enhance the function of natural killer cells resulting in increased tumor destruction and thus increased availability of antigen (43). At the same time, IL-10 has been shown to slow the maturation of dendritic cells thus allowing them to more completely sample the antigen environment before undergoing maturation and trafficking to secondary lymph nodes. In addition, IL-10 can act as a co-stimulatory factor on effector T cells to increase Th1 cytokine expression and to promote the maintenance of CD8+ effector function (44, 45). Taken together, increasing IL-10 and reducing TGF-β may help to positively condition the tumor environment to allow better effector cell activity as well as increased antigen acquisition and presentation at the tumor site.

The results presented here suggest that the membrane-fixed form of TIMP-1 represents an effective version of the protein for use in therapeutic applications directed against melanoma. The data appears to contrast recent publications suggesting a general anti-apoptotic effect of TIMP-1 (14). It is important to note that our studies, the TIMP-1-GPI is not over expressed by the target cell, but rather added to engineer the proteolytic environment of the cell surface. In addition, the agent appears to have unique properties not previously associated with TIMP-1 (18, 19). Based on the chemistry of the GPI-anchor, the TIMP-1 fusion protein is ideally suited to be efficiently incorporated into liposomes. Through the use of thermally sensitive liposomes, the perfusion of TIMP-1-GPI loaded liposomes in the context of regional hyperthermic therapy would allow the targeted delivery and release of the agent into the tumor environment (46-48). Released at the tumor site, TIMP-1-GPI may then synergize with the hyperthermic therapy to reduce tumor growth over multiple mechanisms, including direct effects such as inhibition of tumor cell proliferation and increased sensitivity to apoptosis, as well as indirect effects by releasing immune effector cells from inhibition and fostering their cytotoxic activity through reduction of active TGF-β and increase in IL-10, respectively.

### TIMP proteins and constructs

In the following, a plurality of embodiments relating to the TIMP fusion constructs are described, whereby the constructs were produced and provided for treatment of cancer and as agents in the field of regenerative medicine. In a first embodiment, the TIMP molecule is selected from the group consisting of TIMP-1, TIMP-2, TIMP-3 and TIMP-4, and is preferably linked to an anchor for anchoring one or more than one of the above TIMPs into a cell membrane. In a preferred embodiment this anchor is a GPI.

In another preferred embodiment, the GPI sequence is 36 amino acids in length. A TIMP construct in the sense of the invention is amino acid sequence representing a TIMP protein fused to an anchor for anchoring TIMP into a cell membrane. A TIMP construct can also mean an nucleic acid construct coding for one of the above amino acid sequences sequence representing a TIMP protein which is fused to an anchor for anchoring TIMP into a cell membrane.

In a further embodiment, the TIMP protein that is selected from the group consisting of TIMP-1, TIMP-2, TIMP-3 and TIMP-4 and fused to the GPI sequence. In a preferred embodiment, said TIMP molecule is truncated to the first 50, 50-100 or 50-152 N-terminal amino acid residues. More preferably, the TIMP molecule is truncated to the first 152 N-terminal amino acid residues and is the TIMP-1 molecule. The term "truncated" refers to a TIMP nucleic acid or amino acid sequence that contains less that the full number of nucleic acid bases or amino acid residues found in a native TIMP nucleic acid sequence or protein or to a nucleic acid or amino acid sequence has been deleted of non-desired sequences.)

In yet a further embodiment, the TIMP fusion construct is defined by a sequence selected from the group consisting of SEQ ID NOs: 1, 2, 3, 4 and 5 disclosed in W02007/039109.

The obtained construct can then be expressed in any suitable cell line or host cell to obtain the functional TIMP polypeptide or protein. For this purpose, any of the suitable known vectors or plasmids can be used to express the GPI-anchored TIMP proteins of the present invention.

In a preferred embodiment, and by way of example, one vector used for expression of the fusion constructs of the present invention contains the promoter for human elongation factor 1 alpha followed by a multiple cloning site and an internal ribosomal binding site which allows bicistronic expression of the construct and dihydrofolate reductase (DHFR) used as a selection marker (Mack, et al., P.N.A.S. USA 92:7021, 1995). The 3' end (carboxyl terminal) of the TIMP protein is fused either directly to a GPI-linkage sequence (e.g. derived from lymphocyte function-associated antigen-3 (LFA-3)). The resultant fusion constructs are transfected into dihydrofolate reductase (DHFR)-deficient Chinese hamster ovary (CHO) cells and the selection is performed as described (Mack, et al., P.N.A.S. USA 92:7021-7025, 1995). In a preferred embodiment, the transfectants can be exposed to methotrexate to increase the expression rate by gene amplification.

Although the use of TIMP-1 (Bode & Maskos, 2003) for the preparation of GPI-anchored TIMP protein is preferred in the present invention, the skilled person will recognize that also other TIMP proteins may be used for the practice of the present invention. Further examples of human TIMPs that are useful are TIMP-2, TIMP-3, and TIMP-4 (Mannello F, et al., 2001). The used TIMPs are derived from human sources and administered to treat human cancer cells. The skilled person will likewise recognize that also homologs of TIMP, in particular TIMP-1, in other organisms than human will have a similar effect in killing tumor cells. For example, in some embodiments, the sequence of TIMP-1 derived from an animal such as dog, cat, mouse, rabbit, cow or sheep, and bird may be used for the construction of a TIMP-GPI fusion construct of the present invention. The TIMP-GPI chimera will subsequently be applied to the site of tumor in similar fashion as described for a human individual.

### GPI-Alternatives

GPI is the preferred anchor for anchoring TIMP into a cell membrane. However, the skilled person may consider alternatives to GPI either naturally occurring or artificially designed. Theses GPI alternatives have to be able to be attached to TIMP and their attachment should not result in any change of function compared to the function of a TIMP linked to a GPI. However, an improved function of a GPI alternative, like conferring improved therapeutic efficacy of the TIMP constructs is contemplated. One know GPI alternative is tetradecadienoic acid (US Patent 570259).

### Compositions allowing the heat controlled release of TIMP

A preferred embodiment of the invention is the combination of treatment with a TIMP construct with heat treatment. To allow an even more effective treatment the topical release of TIMP may be triggered by incorporation into heat labile vesicles.

In this way, the vesicles can be introduced into the system of a subject to be treated and do not release the TIMP construct until they have reached an area that is heat treated. An area that is heat treated is an area wherein the temperature is raised above normal body temperature, i.e. 37°C but the temperature remains sublethal, i.e. below or at 42°C. At the area that is heat treated the vesicles disassemble due to the exposure to heat and release the TIMP construct which can subsequently preferentially associate with the heat treated areas. In this way, a topical administration is ensured. The advantages are that the treatment is confined to the area of interest. Thus, a higher concentration of the TIMP construct in the treatment area is ensured, an overall reduced amount of TIMP construct is required, and an undesirable treatment of other areas than the treatment area which may possibly result in side effects is reduced or prevented.

### Treatment of solid cancers with TIMP constructs

The present invention is especially useful for the treatment of solid cancers. Solid cancers appear in the form of breast cancer, prostate cancer, sarcomas, osteosarcoma and melanoma. The TIMP constructs of the invention can be used to treat those solid cancers. In particular, it was found that the TIMP constructs of the invention can be used in combination with hyperthermic conditions to treat solid cancers, in particular osteosarcoma, breast cancer, or melanoma. TIMP constructs can be administered topically or systematically to a patient in need of treatment. For topical administration the TIMP constructs of the invention can be formulated using any of the formulations suitable for topical/local treatment recited below. Such formulation comprising a TIMP construct is then applied to the area of disease comprising the tumor requiring treatment. Together with the application of the TIMP constructs of the invention and/or the formulations comprising said TIMP constructs a hyperthermic treatment can be performed on the area in need of treatment. Hyperthermic treatment can be on the full body or preferentially locally/topically. Topical hyperthermic treatment of the skin or other areas of the body is generally known in the art and can be performed by the use of infrared light, electric blankets, electric probes, water baths, catheters and other devices (see, e.g. US2002193682). The temperature can also be increased of treatment by transporting a heated liquid via a catheter to the region of treatment.

The temperature during the hyperthermic treatment is preferably above the usual body core temperature of 37°C. In another embodiment the temperature is hyperthermic but sublethal for the cells in the affected area. In those cases, the temperature is below 42°C. In a preferred embodiment the temperature is closely monitored by the use of suitable monitoring devices to ensure the maintenance or adjustment of/to the desired temperature. Suitable monitoring devices are known in the art and comprise thermoelectric probes, microprobes, digital or non digital thermometers, infrared cameras or combinations thereof etc.

In a preferred embodiment the TIMP constructs are comprised in the heat labile vesicles discussed above.

The TIMP constructs of the invention can be, when topically applied, administered externally or into the skin cancer. External topical application means to bring the TIMP constructs of the invention into contact with the skin cancer without using any invasive device. This can be done by applying ointments, salves, gels, creams, sprays, etc. It is also appreciated to use invasive devices like in

### Treatment of skin cancer with TIMP constructs

The TIMP constructs of the invention can be used to treat skin cancer. This is surprising since melanoma initially remained largely resistant to treatment with TIMP constructs. It was found here for the first time that a combination of the TIMP constructs of the invention with hyperthermic treatment lead to synergistic anti-tumor effect. TIMP constructs can be administered topically or systematically to a patient in need of treatment. For topical administration the TIMP constructs of the invention can be formulated using any of the formulations suitable for topical/local treatment recited below. Such formulation comprising a TIMP construct is then applied to the area of the skin cancer requiring treatment. It is also envisioned that the formulations of the invention are incorporated into medical bandage or plasters to ensure a sustaining and local impact of the formulations of the invention on the treated skin area. Together with the application of the TIMP constructs of the invention and/or the formulations comprising said TIMP constructs a hyperthermic treatment can be performed on the area in need of treatment. Hyperthermic treatment can be on the full body or preferentially locally/topically. Topical hyperthermic treatment of the skin or other areas of the body is generally known in the art and can be performed by the use of infrared light, electric blankets, electric probes, water baths, catheters and other devices (see, e.g. US2002193682). The temperature can also be increased of treatment by transporting a heated liquid via a catheter to the region of treatment.

The temperature during the hyperthermic treatment is preferably above the usual body core temperature of 37°C. In another embodiment the temperature is hyperthermic but sublethal for the cells in the affected area. In those cases, the temperature is below 42°C. In a preferred embodiment the temperature is closely monitored by the use of suitable monitoring devices to ensure the maintenance or adjustment of/to the desired temperature. Suitable monitoring devices are known in the art and comprise thermoelectric probes, microprobes, digital or non digital thermometers, infrared cameras or combinations thereof etc. In a preferred embodiment the TIMP constructs are comprised in the heat labile vesicles discussed above.

The TIMP constructs of the invention can be, when topically applied, administered externally or into the skin cancer. External topical application means to bring the TIMP constructs of the invention into contact with the skin cancer without using any invasive device. This can be done by applying ointments, salves, gels, creams, sprays, etc. It is also appreciated to use invasive devices like injection needles, catheters, *others?* etc. for applying the TIMP constructs into the skin.

### Formulation of the TIMP constructs and modes of administration

Pharmaceutical compositions based on the TIMP constructs of the present invention may be formulated in a conventional manner using one or more physiologically acceptable carriers or excipients. Techniques and formulations generally may be found in Remmington's Pharmaceutical Sciences, Meade Publishing Co., Easton, Pa. For the purposes of injection, the compounds of the invention can be formulated in a liquid solution, preferably in a physiologically compatible buffer such as Hank's solution or Ringer's solution. In addition, the compounds may be formulated in solid form and redissolved, or suspended immediately prior to use. Lyophilized forms are also suitable.

In addition to these formulations, the compounds may also be formulated as a depot preparation. These long acting depot formulations may be administered by implantation (for example, subcutaneously or intramuscularly) or by injection. Thus, the compounds may be formulated with suitable polymeric or hydrophobic materials (for example, as an emulsion in an acceptable oil) or as an ion exchange resin, or as a sparingly soluble derivative, such as a sparingly soluble salt. Other suitable delivery systems include microspheres, which offer the possibility of a local and noninvasive delivery of drugs over an extended period of time. This particular technology utilizes microspheres having a precapillary size that can be injected via a coronary catheter into any selected part of a tissue, e.g. the heart or other organs, without causing a resulting inflammation or ischemia. The administered therapeutic is slowly released from these microspheres and readily taken up by cells present in the surrounding tissue (e.g. injured or cancerous cells).

For topical administration, the oligomers of the invention can be formulated into ointments, salves, gels, or creams generally known in the art. A wash solution containing the oligomer can be used locally to treat an injury or inflammation or to generally accelerate the healing process.

The TIMP constructs of the present invention may be combined when preparing a medicament, so that the resulting medicament comprises more than one, preferably two, and even more preferably three different TIMP constructs. With this approach, the amplified and novel bioactivities of the different members of the TIMP family may be preferentially combined and targeted to the cell surface, which can lead to a synergistic effect. For example, the TIMP-1 fusion constructs inhibits most MMPs, except MMP-2 and MMP14. Therefore, any of the TIMP-1 constructs may be combined with any of the TIMP-2 or TIMP-4 constructs which both preferentially inhibit MMP-2. Therefore, by means of this combination, a more complete inhibition of the MMP family can be achieved.

In one embodiment, the formulations of the present invention therefore comprise a TIMP-1 construct, or a TIMP-2 and/or a TIMP-4 construct. In a preferred embodiment, the formulation comprises a TIMP-1 construct selected from the group consisting of a truncated TIMP-1-GPI as encoded by SEQ ID NO:1, a truncated TIMP-1-frac-GPI as encoded by SEQ ID NO:5, and a truncated TIMP-1-muc-GPI as encoded by SEQ ID NO:2, and a TIMP-2 and/or a TIMP-4 construct. Preferably, the TIMP-2 construct is encoded by SEQ ID NO:3.

In a further embodiment, the formulation comprises a TIMP-3 construct of the present invention, preferably that encoded by SEQ ID NO:4, which inhibits TACE together with at least one of the TIMP constructs selected from the group consisting of a truncated TIMP-1-GPI as encoded by SEQ ID NO:1, a truncated TIMP-1-frac-GPI, a truncated TIMP-1-muc-GPI, a TIMP-2 and TIMP-4 construct. Preferably, the TIMP-1 and TIMP-2 constructs are encoded by SEQ ID NOs: 1, 2, 3 and 5, respectively.

### Kits of the invention

In addition, kits or systems are provided comprising a TIMP construct of the invention and a heating device. Any of the above described TIMP constructs can be used in a kit of the invention. Heating devices may be bulbs for infrared light, electric blankets, electric probes, and other devices.

### EXAMPLES

### EXAMPLE 1: MATERIAL AND METHODS

### Tumor cell lines and cell culture

The human melanoma cell lines 624.38-MEL, 93.04A12MEL, SK-MEL23, WM115 and WM266-4 were used. The cell lines used represent a spectrum covering diverse melanoma characteristics. 624.38-MEL is a clone of the bulk melanoma cell line 624 expressing high levels of HLA-A2 molecules on the cell surface (21). WM 115 (ESTDAB-066) and WM266-4 (ESTDAB-076) are human melanoma cell lines derived from primary tumor and from metastatic tumor, respectively. The cell line 93.04A12MEL was a gift (Peter Srier, Lieden, Netherlands). SK-MEL23 was obtained from the ATCC and is melanotic while all other lines studied represent amelanotic tumors. Cells were incubated at 37°C in a humidified atmosphere of 5% CO2 and 95% humidity, unless otherwise stated, in medium supplemented with 1% streptomycin sulfate and sodium penicillin G and 1% MEM non-essential amino acids and 10% FCS.

### Purification of TIMP-1-GPI protein

The TIMP-1-GPI protein was produced and purified as previously described (18). Briefly, human TIMP-1 was cloned from cDNA using hTIMP-1 specific primers, fused without a translation stop codon to the GPI-signal sequence cloned from human LFA-3 (17, 22) and subcloned into pEF-DHFR. The expression plasmid was stably introduced into DHFR deficient Chinese hamster ovary (CHO) cells and selected as described (23). TIMP-1-GPI-fusion protein was purified from the CHO cells by Triton X-100 detergent extraction followed by column purification using DEAE, heparin sepharose and size exclusion (18). Phosphatidylserine was used a GPI anchor control (P5660, Sigma Chemical Co).

### Clonogenic assay for survival of heat-treated melanoma cells

The ability of tumor cells to form colonies after hyperthermia exposure was assessed using the clonogenic assay as described (24, 25). The melanoma cells were exposed to 41.8°C or 37°C for 2 h. The cells were then harvested by trypsinization and seeded in duplicate T25 tissue culture flasks at 200 and 500 cells per flask and allowed to form colonies in an undisturbed, humidified, 37°C/5% CO2 air atmosphere. After seven days, the flasks were washed with 0.9% NaCl solution and cell colonies were stained with crystal violet solution (20% ethanol, 0.8% ammonium oxalate and 2% crystal violet). Only colonies containing at least 50 cells were considered to be viable survivors. The number of colonies was counted and compared to the input cell numbers to obtain the percent survival.

### Fluorescence-activated cell sorting (FACS) analysis

Cells were detached with 1.5 mM EDTA (Biochrom A, Berlin, Germany No. L2113) in 1 x PBS and incubated for 60 min on ice with antibodies specific for human TIMP-1 (IM32L) and IgG1□ (SIGMA-ALDRICH, Taufkirchen, Germany No. M9269). Cells were washed three times with 1 x PBS, incubated with FITC-conjugated donkey anti-mouse mAB (DAKO A/S, Glostrup, Denmark No. F0313) for 45 min on ice, then washed three times with 1 x PBS and analyzed using a flow cytometer (FACSCalibur, Becton Dickinson and Company, San Jose, CA, USA) and CellQuest software.

### Incorporation of TIMP-1-GPI into cell membranes

Melanoma cells (5-10 x 106 cells/ml) were incubated with 14 ng/ml of purified hTIMP-1-GPI at 37°C/5% CO2. The cells were then washed three times with cold 1 x PBS and analyzed by FACS using human TIMP-1 specific monoclonal antibodies (see above).

### Proliferation

WM266-4 and SK-MEL23 cells (30 □ 103/100 µl medium) were cultured in 96-well microtiter plates for 24 h under standard conditions to yield firmly attached and stably growing cells. After discarding supernatants, 50 µl of medium containing TIMP-1-GPI, buffer, denatured TIMP-1-GPI, phosphatidylserine or rhTIMP-1 was added to the cells and incubated for 24 to 72 h. Additional plates were treated in parallel or in combination with hyperthermic stress for 2 hours at 41.8°C. Then 50 µl of a 1 mg/ml solution of (3,5- Dimethylthiazol-2-yl] - 2,5 - diphenyl-tetrazolium bromide) MTT (SIGMA-ALDRICH, Taufkirchen, Germany No. M2128) was added. After a 3-h incubation at 37°C, formazan crystals were dissolved by addition 100 µl isopropanol and 0.04 N HCl. Absorbance was then measured at 550 nm using GENios plus TECAN ELISA reader. Cell proliferation was calculated as previously described (6). For each experiment at least 6-wells were analyzed per experimental condition and time point. A minimum of six wells were analyzed per experimental condition and time point and are presented as mean values with standard deviation.

### Zymography

WM266-4 and SK-MEL23 cells were cultured in 24 wells plate (5 □ 104 cells/well) to subconfluency. The medium was exchanged for 24 h with serum-free medium containing either rhTIMP-1 or increasing amounts of TIMP-1-GPI and incubated for 24 h, 48 h and 72 h. Cell supernatants were then analyzed by gelatin zymography using 10% SDS-polyacrylamide gels (Invitrogen, Groningen, Netherlands, No. EC61755BOX) as described (18). Recombinant MMP-9 protein (Amersham Biosciences, Uppsala, Sweden, No. RPN2634) was used as positive signal on the gel.

### FAS detection

FAS expression on the surface of melanoma lines was assessed by flow cytometry using a non-activating anti-FAS mAB (L-958, was a gift from H. Engelmann, Munich). To induce FAS expression, untreated cells and cells treated for 72 h with 14 ng/ml TIMP-1-GPI, denatured TIMP-1-GPI or rhTIMP-1 protein either with, or without hyperthermic exposure (2 h at 41.8°C) were -detached with 1.5 mM EDTA and stained with L-958 and analyzed by flow cytometry.

### FAS induced apoptosis and annexin-V-detection

Detection and quantification of apoptotic vs. necrotic cells at the single cell level was performed using annexin-V-FLUOS staining Kit (Becton, Dickinson and Company, Heidelberg, Germany, No. 556547). Melanoma cells were seeded at 1 x 106 cells/well into 24-well plates and allowed to attach over night. The wells were then rinsed 3 times with 1 x PBS and 1 ml of serum-free RPMI 1640 medium was added, followed by 14 ng/ml of TIMP-1-GPI, denatured TIMP-1-GPI or rhTIMP-1. Cells were incubated for 24 h at 37°C/5% CO2. After 72 h, 1 µg/ml anti-FAS activating mAB L-957 (26) or isotype control were added and the cells were further incubated for 24 h at 37°C/5% CO2. The cells were then washed with 1 x PBS, gently pelleted and resuspended in staining solution (annexin-V-fluorescein labeling reagent and propidium iodide (PI) in Hepes buffer) for 15 min at room temperature. The cells were then analyzed by flow cytometry. A time-course study showed that annexin-V binding to melanoma cells precedes PI reactivity.

### Western Blot analyses

Western blot was used for the detection of Bcl-XL (Santa Cruz Biotechnology, INC, Santa Cruz, California USA No. SC-7195) Bcl-2, (ALX-804-225), Bax (ANC-357-040), Apaf-1, (Stressgen, Ann Arbor, USA, No. AAP-300) and □-actin (Acris Hiddenhausen, Germany, No. ab8227). The anti-Hsp70 monoclonal rat IgG1 antibody 6B3 was a gift (E. Kremmer, Munich) (27). The cells were washed three times with cold 1 x PBS and detached with 1.5 mM EDTA. The cells were rotated 1 h at 4 °C with hypertonic lysis buffer (5 mM Tris, 2 mM MgCl, 0.1 mM EDTA, 1 mM PMSF (Roche, Basel, Switzerland No. 236608), 2 µg/ml aprotinin (Roche, Basel, Switzerland No. 1583794) 2 µg/ml leupeptin (Sigma, Taufkirchen, Germany No. L2884), 1 µg/ml pepstatin A (Roche, Basel, Switzerland No.1524488), pH 7.4). Cell membranes were isolated with extraction buffer (100 mM NaCl, 1 % Triton X-100, 10 mM Tris, 5 mM EDTA, 1 mM PMSF 2 µg/ml Aprotinin, 2 µg / ml leupeptin, 1 µg / ml pepstatin A, pH 7.4) followed by centrifugation at 11,000g for 20 min at 4 °C. The supernatant was used for further Western blot tests.

Whole-cell lysates containing 40 µg protein, as determined by the Lowry method (BioRad, Munich, Germany), were denatured by boiling for 5 min in SDS sample buffer, loaded onto one lane and separated by 4-20 %SDS-Page electrophoresis. After electrophoresis, proteins were transferred to PVDF membranes (Invitrogen, Groningen, Netherlands. No. LC2002) and probed with appropriate antibodies. Specific proteins were detected using a commercial Western blot analysis kit, Chemiluminescent Immunodetection System (Invitrogen, Groningen, Netherlands).

### ELISA for TGF-β and IL-10 proteins

WM266-4 and SK-MEL23 cells were cultured in 24 wells plate (5 □ 104 cells/well). The medium was exchanged for 24 h with serum-free medium containing either rhTIMP-1 or increasing TIMP-1-GPI and incubated for 1 h. The cells were washed and a portion of the cells were exposed to 41.8°C for 2 h. Then the cells were again washed with 1 x PBS and incubated again in serum free culture media for an additional 72 h at 37°C in a humidified 5% CO2 atmosphere. The supernatant were harvested and used for ELISA. The TGF-β and IL-10 concentrations were measured using commercial ELISA kits from BD Biosciences (San Jose, CA USA, No. 559119 for TGF-β and No. 555157 for IL-10). TGF-β

### EXAMPLE 2: Incorporation of exogenously added TIMP-1-GPI into the surface membrane of melanoma cell lines

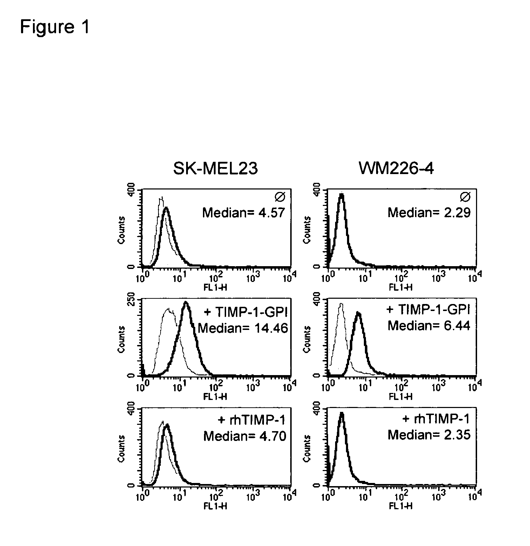

GPI-anchored TIMP-1 protein was generated and isolated as previously described (18, 19). The incorporation of purified GPI-anchored TIMP-1 protein into the surface membranes of the tumor cells was demonstrated using two exemplary melanoma cell lines, SK-MEL23 and WM266-4, following the incubation of the cell lines with 14 ng/ml of purified TIMP-1-GPI or recombinant human (rh)TIMP-1 control protein for one h at 37°C. Surface associated TIMP-1 protein was detected using FACS and an anti-human TIMP-1 monoclonal antibody. Addition of control rhTIMP-1 did not lead to detectable TIMP-1 on the cell surface while GPI-anchored TIMP-1 resulted in a surface signal for TIMP-1 (Figure 1). The GPI anchored TIMP-1 protein could be efficiently cleaved from the surface of the melanoma cells following treatment with phospholipase C (data not shown) (18, 19).

### EXAMPLE 3: The effect of TIMP-1-GPI treatment in conjunction with thermal stress on the survival and proliferation of melanoma cells

The survival capacity of cells in the context of heat treatment is commonly measured using a clonogenic assay (6). In this procedure, the cells are exposed to a specific thermal dose represented by a defined period of time and temperature. We had previously established the thermal sensitivity of melanoma cell lines (6). By clonogenic assay, a temperature of 41.8°C delivered for an interval of two h was found to represent a sub-lethal thermal dose (6). For subsequent experiments this thermal dose was used as a basis to study the potential additive effects of TIMP-1-GPI treatment in the context of thermal treatment.

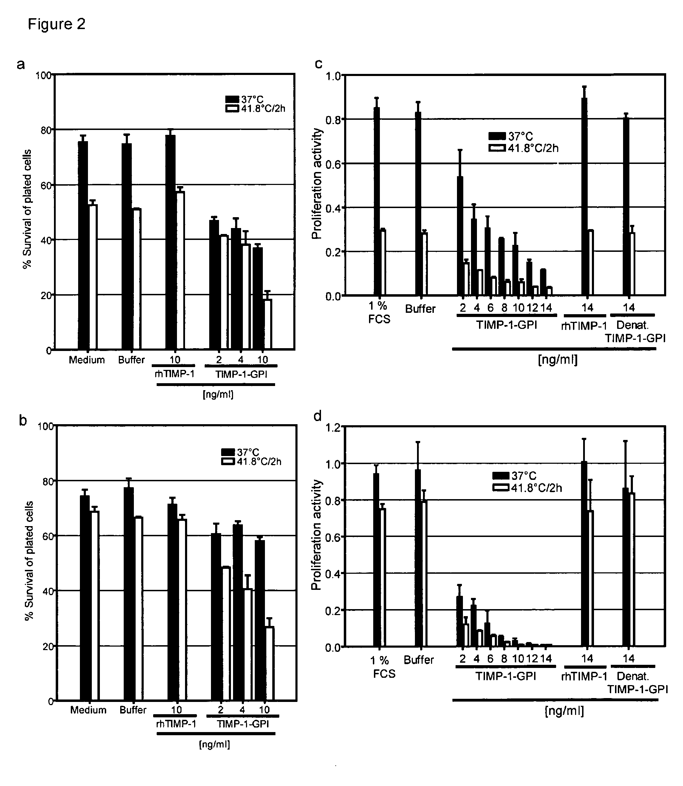

The cumulative effect of the sub-lethal thermal dose (41.8°C for two hours) with TIMP-1-GPI treatment was then evaluated using SK-MEL23 and WM266-4. The cell lines were treated with 14 ng/ml TIMP-1-GPI for one h, washed, and subjected to either hyperthermic stress or physiologic temperature (37°C). In Figure 2a and b the resultant survival data at 37°C and 41.8°C in the context of TIMP-1-GPI and control rhTIMP-1 are depicted. The figures represent the number of surviving colonies from 200 melanoma cells following treatment. TIMP-1-GPI, but not rhTIMP-1 was found to reduce the clonogenic survival of SK-MEL23 melanoma cells, and to a lesser extent WM226-4. Hyperthermic stress in combination with TIMP-1-GPI further reduced the survival capacity of all melanoma cell lines tested.

We have previously shown that treatment with TIMP-1-GPI can either enhance or suppress cell proliferation depending on the specific cell type studied (18, 19). MTT assays were performed to assess the effect of TIMP-1 surface engineering on the proliferation of melanoma cells. The exogenously added TIMP-1-GPI protein was found to elicit a dose-dependent decrease in proliferation of SK-MEL23 and WM226-4 at 24 h (Figure 2c and d). In contrast, heat denatured TIMP-1-GPI and rhTIMP-1 had no effect on proliferation. Additional controls using an equal molar concentration of phosphoinositol did not alter proliferation of the cells (data not shown) (19). The sublethal hyperthermic dose also reduced cell proliferation while the combined TIMP-1-GPI/hyperthermic treatment amplified the inhibition seen with TIMP-1-GPI alone.

### EXAMPLE 4: TIMP-1-GPI protein blocks release of proMMP-2 and proMMP-9 from melanoma cells

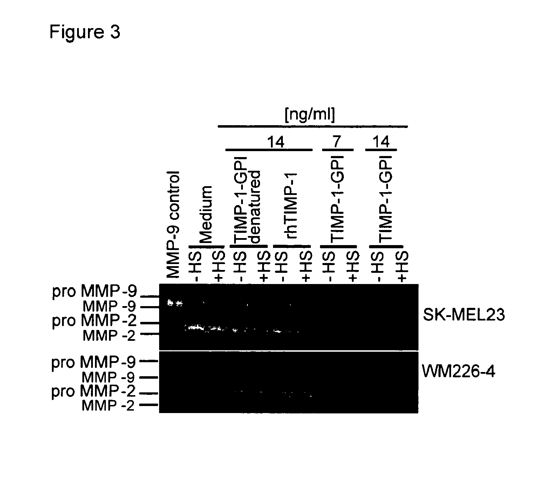

The gelatinases MMP-2 and MMP-9 are thought to play a role the general aggressiveness of tumors including melanoma (28, 29). Gelatinase zymography was used to examine the release of MMP-2 and MMP-9 into the culture medium from the melanoma cells. SK-MEL23 and WM226-4 cell lines constitutively secrete both proMMP-2 and proMMP-9 (Figure 3a and b). The effect of TIMP-1-GPI on the release of MMP-2 and MMP-9 proteins was tested in response to two concentrations of TIMP-1-GPI (7 ng/ml and 14 ng/ml) and in the context of thermal stress (41.8°C for 2 h). As previously reported for renal cell carcinoma (19), treatment with TIMP-1-GPI effectively blocked the secretion of both gelantinases. Control rhTIMP-1 protein at 14 ng/ml had no effect on proMMP-2 or proMMP-9 secretion. Thermal treatment did not significantly influence the release of the gelatinases into the growth media with or without TIMP-1-GPI treatment.

### EXAMPLE 5: TIMP-1-GPI and heat shock treatment renders melanoma sensitive to FAS-mediated killing

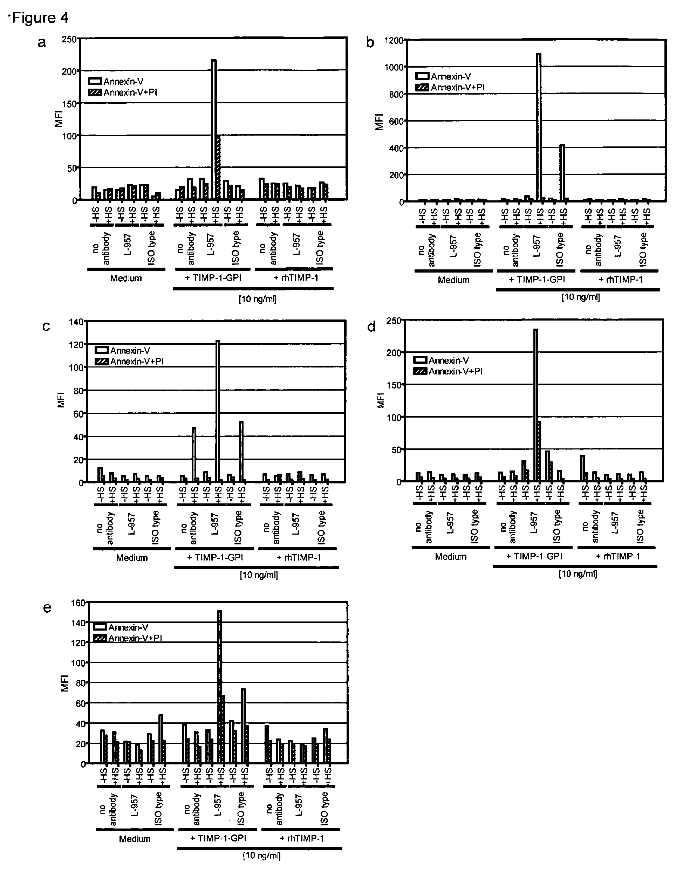

Melanoma is generally resistant to induced apoptosis (4, 5, 7). The level of FAS surface expression on SK-MEL23, WM226-4, WM115, 624.38 MEL and 93.04A12 MEL cell lines was assessed by flow cytometry using a non-activating anti-FAS mAB (L-958). Untreated cells as well as cells treated with 14 ng/ml TIMP-1-GPI or rhTIMP-1 control protein for 72 h were stained with L-958 and analyzed for surface FAS expression. Except for the 624.38 MEL cell line, FAS-protein was found to be expressed by the other four melanoma lines. Treatment with TIMP-1-GPI or rhTIMP-1 had little or no effect on cell surface FAS-expression (Figure 4a). Thermal treatment alone showed a moderate effect on surface FAS expression. The combined treatment of TIMP-1-GPI and hyperthermic treatment resulted in an additive increase in FAS surface expression (Figure 4a).

We have previously demonstrated that the treatment of renal cell carcinoma (RCC) with TIMP-1-GPI can render RCC more sensitive to FAS/CD95-ligation-mediated apoptosis (19). The anti-FAS mAB L-957 can mimic CD95 induced apoptosis in FAS-expressing cells (26). Staining with antibody directed against annexin V-fluoroisothiocyanate (FITC) and incorporation of propidium iodide (PI) into melanoma cells after treatment with L-957 for an additional 24 h was used to detect apoptosis using flow cytometry. The effect of thermal treatment in combination with TIMP-1-GPI on FAS induced apoptosis was assessed in parallel. As demonstrated in Figure 4b-f, in keeping with previous reports, the melanoma cells were found to be largely resistant to FAS-mediated apoptosis before TIMP-1-GPI or hyperthermic treatment. The apoptotic response of the various melanoma cell lines to TIMP-1-GPI or hyperthermic treatment alone was variable, and depended on the individual cell line tested. However, when the cell lines were treated by a combination of TIMP-1-GPI with thermal stress, the FAS inducing antibody (L-957) lead to a synergistic increase in sensitivity to FAS mediated killing in each of the cell lines tested.

### EXAMPLE 6: TIMP-1-GPI and thermal treatment of melanoma cell moderates the expression of apoptotic proteins

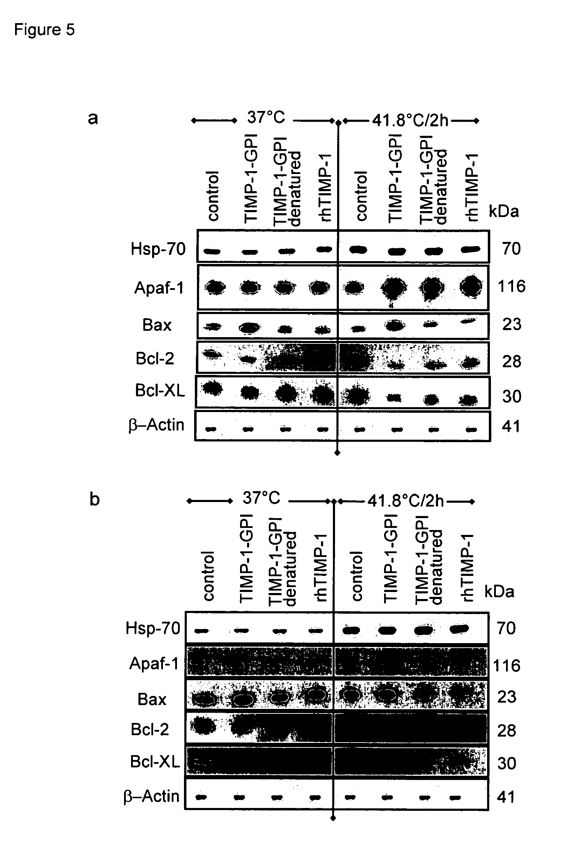

The BCL-2 family of proteins are involved in the regulation of apoptosis (reviewed in (30)). Apoptosis protease-activating factor-1 (Apaf-1) is a key regulator of the mitochondrial apoptotic pathway (31). We have previously shown for renal cell carcinoma that TIMP-1-GPI can alter the expression of pro and anti apoptotic proteins (19). The effect of TIMP-1-GPI and thermal treatment on the protein expression of Bcl-2, Bcl-XL, Bax and Apaf-1 was evaluated. Following a 72 h preincubation period with 14 ng/ml of TIMP-1-GPI or rhTIMP-1 control, with or without hyperthermic treatment, the two exemplary melanoma cell lines SK-MEL23 and WM226-4 were stimulated with 1 µg/ml L-957 (or control mAB) for an additional 24 h. The level of Bcl-2, Bcl-XL, Bax and Apaf-1 proteins were determined using Western blot and specific antibody reagents. In both cell lines, treatment with TIMP-1-GPI lead to an increase in expression of pro-apoptotic Bax and decreased expression of anti-apoptotic Bcl-2 and Bcl-XL proteins. Levels of Apaf-1 were largely unaffected by TIMP-1-GPI treatment (Figure 5a and b). The effect of thermal dose augmented the general increase or decrease in Bcl-2 protein expression and increased Apaf-1 expression in combination with TIMP-1-GPI. Antibody directed against human Hsp70 verified an increase in protein in response to hyperthermic stress (Figure 5a and b).

### EXAMPLE 7: Downregulation of active TGF-β and induced IL-10 secretion by combined TIMP-1-GPI and hyperthermic treatment

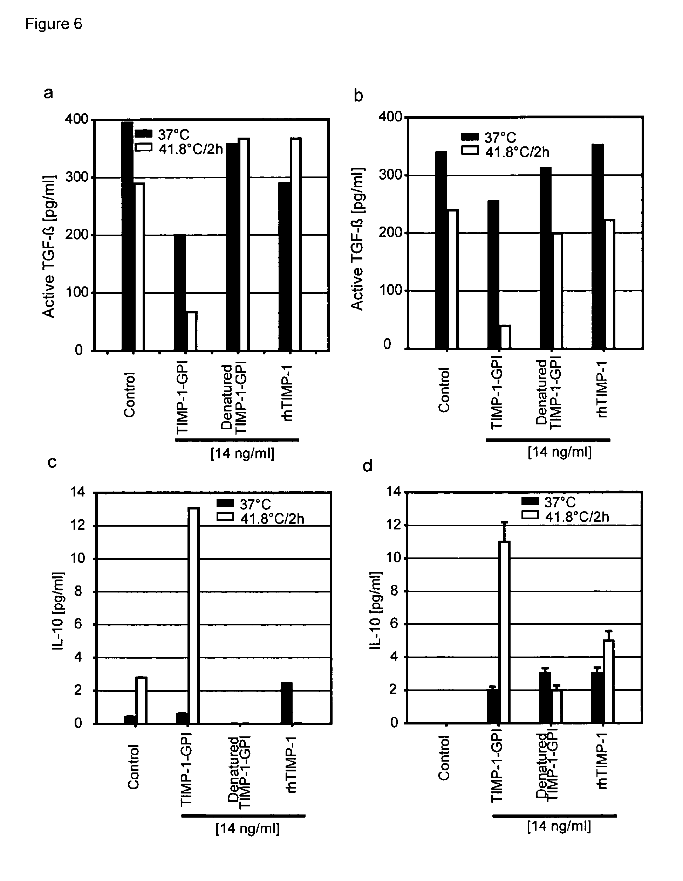

The cytokine TGF-β has been proposed to have a regulatory effect on melanoma biology (32). TGF-β is processed from a latent to active form by the actions of various proteins including MMPs (33, 34). To investigate the effect of TIMP-1-GPI and heat shock on the activation of TGF-β from the latent to active form, total TGF-β protein was compared to that of active TGF-β using specific ELISA on the culture supernatants from SK-MEL23 and WM226-4. Hyperthermic stress or TIMP-1-GPI treatment alone reduced the ratio of active TGF-β to total protein to some extent, however, the combined treatment strongly reduced expression suggesting a combined effect of TIMP-1-GPI and hyperthermic treatment on the cleavage of the pro form of the cytokine to the active protein (Figure 6a and b).

The immunogenicity of melanoma has been linked to expression of the regulatory cytokine IL-10 (35). Untreated melanoma cells were found to secret little IL-10 and the secretion was largely unaffected by either hyperthermic or TIMP-1-GPI treatment. Interestingly, the two modalities appear to act synergistically to increase the secretion of IL-10 protein by the treated SK-MEL23 and WM226-4 cell lines (Figure 8c and d).

### EXAMPLE 8: TIMP-2-GPI, TIMP-3-GPI, or TIMP-4-GPI treatment results in the same cellular effects as for TIMP-1-GPI

Vectors for TIMP-2-GPI, TIMP-3-GPI, and TIMP-4-GPI (hereafter designated TIMP-(2-4)-GPI) were essentially constructed in the same way as having being described for TIMP-1-GPI above. It was found that treatment with either of TIMP-(2-4)-GPI in conjunction with thermal stress has the same effect on the survival and proliferation of melanoma cells as TIMP-1-GPI and that each of them moderates the expression of apoptotic proteins in the same manner as TIMP-1-GPI. It was further found that either of TIMP-(2-4)-GPI in combination with hyperthermic treatment downregulate active TGF-β and induce IL-10 in the same manner as TIMP-1-GPI (data not shown). This is not surprising, since the various TIMP proteins share a high homology and it is widely accepted that these TIMP proteins make up a protein family whose members share a vast number of structural features and are largely interchangeable in their capabilities as inhibitors of MMPs which has been associated with FAS-mediated apoptosis.

### EXAMPLE 9: In vitro release of TIMP-1-GPI using temperature-sensitive liposomes

Liposomes were prepared as described elsewhere (Lindner et al. "Novel temperature-sensitive liposomes with prolonged circulation time", Clin. Cancer Res. 2004 Mar 15;10(6):2168-78) using a 14 ng/ml solution of TIMP-1-GPI in PBS. This solution is applied to SK-MEL23, WM226-4, WM115, 624.38 MEL and 93.04A12 MEL cell lines as in Example 5 and liposomes without TIMP-1-GPI serve as control. As an additional control another set of cell lines SK-MEL23, WM226-4, WM115, 624.38 MEL and 93.04A12 MEL is treated both with and without TIMP-1-GPI but does not receive hyperthermic treatment. The experimental set of cell lines is transferred to an incubator providing the same conditions as the incubator for the cells receiving not hyperthermic treatment but having been adjusted to 42°C. The cells are incubated at 42°C before 5, 15, 30, and 45 min before being processed as desribed in Example 5.

### EXAMPLE 10: In vivo testing of temperature-sensitive liposomes comprising TIMP-1-GPI

Animal studies are performed as described elsewhere (Lindner et al. "Novel temperature-sensitive liposomes with prolonged circulation time", Clin. Cancer Res. 2004 Mar 15;10(6):2168-78). In short, male Syrian golden hamsters are implanted with a dense cell suspension (2 x 10⁵ cells) of A-Mel-3 and the tumor cells are allowed to grow for 1 week. Then, 15.6 µmol lipid/kg body weight or 25 µmol lipid/kg body weight of the liposome preparation (comprising/not comprising TIMP-1-GPI) are infused into the animals and a set of animals receives hyperthermic treatment at 42°C for 1 hour. The tumors are removed from all animal, histological sectioned and analysed with hematoxyline/eosine.

### REFERENCES

1. Fraker DL. Management of in-transit melanoma of the extremity with isolated limb perfusion. Curr Treat Options Oncol 2004;5: 173-84.
2. Sanki A, Kam PC, Thompson JF. Long-term results of hyperthermic, isolated limb perfusion for melanoma: a reflection of tumor biology. Ann Surg 2007;245: 591-6.
3. Garbe C, Eigentler TK. Diagnosis and treatment of cutaneous melanoma: state of the art 2006. Melanoma Res 2007;17: 117-27.
4. La Porta CA. Drug resistance in melanoma: new perspectives. Curr Med Chem 2007;14: 387-91.
5. Facchetti F, Previdi S, Ballarini M, Minucci S, Perego P, La Porta CA. Modulation of pro- and anti-apoptotic factors in human melanoma cells exposed to histone deacetylase inhibitors. Apoptosis 2004;9: 573-82.
6. Milani V, Frankenberger B, Heinz O, et al. Melanoma-associated antigen tyrosinase but not Melan-A/MART-1 expression and presentation dissociate during the heat shock response. Int Immunol 2005.
7. Soengas MS, Lowe SW. Apoptosis and melanoma chemoresistance. Oncogene 2003;22: 3138-51.
8. Egeblad M, Werb Z. New functions for the matrix metalloproteinases in cancer progression. Nat Rev Cancer 2002;2: 161-74.
9. Itoh Y, Nagase H. Matrix metalloproteinases in cancer. Essays Biochem 2002;38: 21-36.
10. Bode W, Maskos K. Structural basis of the matrix metalloproteinases and their physiological inhibitors, the tissue inhibitors of metalloproteinases. Biol Chem 2003;384: 863-72.
11. Nagase H, Woessner JF, Jr. Matrix metalloproteinases. J Biol Chem 1999;274: 21491-4.
12. Brew K, Dinakarpandian D, Nagase H. Tissue inhibitors of metalloproteinases: evolution, structure and function. Biochim Biophys Acta 2000;1477: 267-83.
13. Klier CM, Nelson EL, Cohen CD, Horuk R, Schlondorff D, Nelson PJ. Chemokine-Induced secretion of gelatinase B in primary human monocytes. Biol Chem 2001;382: 1405-10.
14. Sorensen NM, Bystrom P, Christensen IJ, et al. TIMP-1 is significantly associated with objective response and survival in metastatic colorectal cancer patients receiving combination of irinotecan, 5-fluorouracil, and folinic acid. Clin Cancer Res 2007;13: 4117-22.
15. Brand K. Cancer gene therapy with tissue inhibitors of metalloproteinases (TIMPs). Curr Gene Ther 2002;2: 255-71.
16. Chirco R, Liu XW, Jung KK, Kim HR. Novel functions of TIMPs in cell signaling. Cancer Metastasis Rev 2006;25: 99-113.
17. Medof ME, Nagarajan S, Tykocinski ML. Cell-surface engineering with GPI-anchored proteins. Faseb J 1996;10: 574-86.
18. Djafarzadeh R, Mojaat A, Vicente AB, von Luttichau I, Nelson PJ. Exogenously added GPI-anchored tissue inhibitor of matrix metalloproteinase-1 (TIMP-1) displays enhanced and novel biological activities. Biol Chem 2004;385: 655-63.
19. Djafarzadeh R, Noessner E, Engelmann H, et al. GPI-anchored TIMP-1 treatment renders renal cell carcinoma sensitive to FAS-meditated killing. Oncogene 2006;25: 1496-508.
20. Brown O, Cowen RL, Preston CM, Castro MG, Lowenstein PR. Subcellular post-transcriptional targeting: delivery of an intracellular protein to the extracellular leaflet of the plasma membrane using a glycosyl-phosphatidylinositol (GPI) membrane anchor in neurons and polarised epithelial cells. Gene Ther 2000;7: 1947-53.
21. Rivoltini L, Barracchini KC, Viggiano V, et al. Quantitative correlation between HLA class I allele expression and recognition of melanoma cells by antigen-specific cytotoxic T lymphocytes. Cancer Res 1995;55: 3149-57.
22. Kirby AC, Hill V, Olsen I, Porter SR. LFA-3 delta D2: a novel in vivo isoform of lymphocyte function-associated antigen 3. Biochem Biophys Res Commun 1995;214: 200-5.
23. Mack M, Riethmuller G, Kufer P. A small bispecific antibody construct expressed as a functional single-chain molecule with high tumor cell cytotoxicity. Proc Natl Acad Sci U S A 1995;92: 7021-5.
24. Overgaard J, Suit HD. Time-temperature relationship th hyperthermic treatment of malignant and normal tissue in vivo. Cancer Res 1979;39: 3248-53.
25. Issels RD, Nagele A. Influence of thiols on thermosensitivity of mammalian cells in vitro. Methods Enzymol 1990;186: 696-708.
26. Schwabe RF, Hess S, Johnson JP, Engelmann H. Modulation of soluble CD40 ligand bioactivity with anti-CD40 antibodies. Hybridoma 1997;16: 217-26.
27. Noessner E, Gastpar R, Milani V, et al. Tumor-derived heat shock protein 70 peptide complexes are cross-presented by human dendritic cells. J Immunol 2002;169: 5424-32.
28. Ko HM, Kang JH, Jung B, et al. Critical role for matrix metalloproteinase-9 in platelet-activating factor-induced experimental tumor metastasis. Int J Cancer 2007;120: 1277-83.
29. Qu XJ, Yuan YX, Tian ZG, et al. Using caffeoyl pyrrolidine derivative LY52, a potential inhibitor of matrix metalloproteinase-2, to suppress tumor invasion and metastasis. Int J Mol Med 2006;18: 609-14.
30. Igney FH, Krammer PH. Death and anti-death: tumour resistance to apoptosis. Nat Rev Cancer 2002;2: 277-88.
31. Bao Q, Shi Y. Apoptosome: a platform for the activation of initiator caspases. Cell Death Differ 2007;14: 56-65.
32. Rothhammer T, Poser I, Soncin F, Bataille F, Moser M, Bosserhoff AK. Bone morphogenic proteins are overexpressed in malignant melanoma and promote cell invasion and migration. Cancer Res 2005;65: 448-56.
33. Karsdal MA, Larsen L, Engsig MT, et al. Matrix metalloproteinase-dependent activation of latent transforming growth factor-beta controls the conversion of osteoblasts into osteocytes by blocking osteoblast apoptosis. J Biol Chem 2002;277: 44061-7.
34. Illman SA, Lehti K, Keski-Oja J, Lohi J. Epilysin (MMP-28) induces ¶eta mediated epithelial to mesenchymal transition in lung carcinoma cells. J Cell Sci 2006;119: 3856-65.
35. Polak ME, Borthwick NJ, Gabriel FG, et al. Mechanisms of local immunosuppression in cutaneous melanoma. Br J Cancer 2007;96: 1879-87.
36. Barnhart BC, Lee JC, Alappat EC, Peter ME. The death effector domain protein family. Oncogene 2003;22: 8634-44.
37. Jaattela M. Multiple cell death pathways as regulators of tumour initiation and progression. Oncogene 2004;23: 2746-56.
38. Shi Y. Mechanical aspects of apoptosome assembly. Curr Opin Cell Biol 2006;18: 677-84.
39. Gorelik L, Flavell RA. Transforming growth factor-beta in T-cell biology. Nat Rev Immunol 2002;2: 46-53.
40. Gold LI. The role for transforming growth factor-beta (¶eta) in human cancer. Crit Rev Oncog 1999;10: 303-60.
41. Peng Y, Gorelik L, Laouar Y, Li MO, Flavell RA. TGFbeta-mediated immunoregulation. Ernst Schering Res Found Workshop 2006: 155-60.
42. Mocellin S, Panelli MC, Wang E, Nagorsen D, Marincola FM. The dual role of IL-10. Trends Immunol 2003;24: 36-43.
43. Conti P, Kempuraj D, Kandere K, et al. IL-10, an inflammatory/inhibitory cytokine, but not always. Immunol Lett 2003;86: 123-9.
44. Lauw FN, Pajkrt D, Hack CE, Kurimoto M, van Deventer SJ, van der Poll T. Proinflammatory effects of IL-10 during human endotoxemia. J Immunol 2000;165: 2783-9.
45. Santin AD, Hermonat PL, Ravaggi A, et al. Interleukin-10 increases Th1 cytokine production and cytotoxic potential in human papillomavirus-specific CD8(+) cytotoxic T lymphocytes. J Virol 2000;74: 4729-37.
46. Sharma D, Chelvi TP, Kaur J, Ralhan R. Thermosensitive liposomal taxol formulation: heat-mediated targeted drug delivery in murine melanoma. Melanoma Res 1998;8: 240-4.
47. Han HD, Choi MS, Hwang T, et al. Hyperthermia-induced antitumor activity of thermosensitive polymer modified temperature-sensitive liposomes. J Pharm Sci 2006;95: 1909-17.
48. Minko T, Pakunlu RI, Wang Y, Khandare JJ, Saad M. New generation of liposomal drugs for cancer. Anticancer Agents Med Chem 2006;6: 537-52.

## Claims

1. Use of a construct, wherein said construct comprises a tissue matrix metalloproteinase (TIMP) linked to an anchor for anchoring TIMP into a cell membrane for the treatment of a solid tumor.

2. Use of claim 1, wherein the tumor is skin cancer, osteosarcoma or breast cancer.

3. Use of claim 2 wherein the skin cancer is a melanoma.

4. Use of the above claims, wherein the anchor is glycosylphosphatidylinositol (GPI).

5. Use of the above claims, wherein TIMP is selected from the group consisting of TIMP-1, TIMP-2, TIMP-3, or TIMP-4.

6. Use of the above claims, wherein the TIMP is to be administered in vesicles

7. Use of claim 6, wherein the vesicles are heatlabile.

8. Use of the above claims, wherein the constructs is to be administered together with hyperthermic treatment.

9. Use of claim 8, wherein the hyperthermic treatment is to be performed with a sub lethal thermal dose.

10. Use wherein the sub lethal thermal dose is to be administered at a temperature of between 40 and 43 degree centigrade.

11. Use of claim 10 wherein the temperature is between 41 and 42 degree centigrade or is 41.8 degree centrigade.

12. Use wherein the thermal dose is to be administered for 0.5 to 10 hours, for 0.5 to 8 hours, for 1 to 6 hours, for 1 to 4 hours, or for 2 hours.

13. Composition comprising TIMP linked to an anchor for anchoring the TIMP into a cell membrane and a heat labile vesicle.

14. Kit comprising TIMP linked to an anchor for anchoring the TIMP into a cell membrane and a heating device.

15. The kit further comprising a heat labile vesicle.


SEQUENCE LISTING

<110>  Nelson, Peter Jon

<120>  TREATMENT OF SOLID TUMORS WITH TISSUE INHIBITORS OF
       METALLOPROTEINASES (TIMPS)

<130>  109-004

<160>  5

<170>  PatentIn version 3.3

<210>  1
<211>  582
<212>  DNA
<213>  Artificial Sequence

<220>
<223>  Truncated human TIMP-1 fused to GPI sequence

<400>  1
gaattcatgg ccccctttga gcccctggct tctggcatcc tgttgttgct gtggctgata      60

gcccccagca gggcctgcac ctgtgtccca ccccacccac agacggcctt ctgcaattcc     120

gacctcgtca tcagggccaa gttcgtgggg acaccagaag tcaaccagac caccttatac     180

cagcgttatg agatcaagat gaccaagatg tataaagggt tccaagcctt aggggatgcc     240

gctgacatcc ggttcgtcta cacccccgcc atggagagtg tctgcggata cttccacagg     300

tcccacaacc gcagcgagga gtttctcatt gctggaaaac tgcaggatgg actcttgcac     360

atcactacct gcagttttgt ggctccctgg aacagcctga gcttagctca gcgccggggc     420

ttcaccaaga cctacactgt tggctgtgag gaatgcacag tgtctagaac aacctgtatc     480

ccaagcagcg gtcattcaag acacagatat gcacttatac ccataccatt agcagtaatt     540

acaacatgta ttgtgctgta tatgaatgta ttatgagtcg ac                        582

<210>  2
<211>  900
<212>  DNA
<213>  Artificial Sequence

<220>
<223>  Truncated human TIMP-1 fused to mucin domain and GPI sequence

<400>  2
gaattcatgg ccccctttga gcccctggct tctggcatcc tgttgttgct gtggctgata      60

gcccccagca gggcctgcac ctgtgtccca ccccacccac agacggcctt ctgcaattcc     120

gacctcgtca tcagggccaa gttcgtgggg acaccagaag tcaaccagac caccttatac     180

cagcgttatg agatcaagat gaccaagatg tataaagggt tccaagcctt aggggatgcc     240

gctgacatcc ggttcgtcta cacccccgcc atggagagtg tctgcggata cttccacagg     300

tcccacaacc gcagcgagga gtttctcatt gctggaaaac tgcaggatgg actcttgcac     360

atcactacct gcagttttgt ggctccctgg aacagcctga gcttagctca gcgccggggc    420

ttcaccaaga cctacactgt tggctgtgag gaatgcacag tgtctagact tgatctcaaa    480

gaatgtggac atgcttactc ggggattgtg gcccaccaga agcatttact tcctaccagc    540

cccccaactt ctcaggcctc agagggggca tcttcagata tccacacccc tgcccagatg    600

ctcctgtcca ccttgcagtc cactcagcgc cccaccctcc cagtaggatc actgtcctcg    660

gacaaagagc tcactcgtcc caatgaaacc accattcaca ctgcgggcca cagtctggca    720

gttgggcctg aggctgggga gaaccagaag cagccggaaa aaaatgctgg tcccacagcc    780

tctagcacaa cctgtatccc aagcagcggt cattcaagac acagatatgc acttataccc    840

ataccattag cagtaattac aacatgtatt gtgctgtata tgaatgtatt atgagtcgac    900

<210>  3
<211>  599
<212>  DNA
<213>  Artificial Sequence

<220>
<223>  Human TIMP-2 fused to GPI sequence

<400>  3
gaattcatgg caaccctaga gaggatccag tatgagatca agcagataaa gatgttcaaa    60

gggcctgaga aggatataga gtttatctac acggccccct cctcggcagt gtgtggggtc    120

tcgctggacg ttggaggaaa gaaggaatat ctcattgcag gaaaggccga gggggacggc    180

aagatgcaca tcaccctctg tgacttcatc gtgccctggg acaccctgag caccacccag    240

aagaagagcc tgaaccacag gtaccagatg ggctgcgagt gcaagatcac gcgctgcccc    300

atgatcccgt gctacatctc ctccccggac gagtgcctct ggatggactg ggtcacagag    360

aagaacatca acgggcacca ggccaagttc ttcgcctgca tcaagagaag tgacggctcc    420

tgtgcgtggt accgcggcgc ggcgcccccc aagcaggagt ttctcgacat cgaggaccca    480

tctagaacaa cctgtatccc aagcagcggt cattcaagac acagatatgc acttataccc    540

ataccattag cagtaattac aacatgtatt gtgctgtata tgaatgtatt atagtcgac    599

<210>  4
<211>  758
<212>  DNA
<213>  Artificial Sequence

<220>
<223>  Mutated human TIMP-3 fused to GPI sequence

<400>  4
gaattcatga ccccttggct cgggctcatc gtgctcctgg gcagctggag cctgggggac    60

tggggcgccg aggcgtgcac atgctcgccc agccaccccc aggacgcctt ctgcaactcc    120

gacatcgtga tcgcggccgc ggtggtgggg gcgaagctgg tagcggaggg gcccttcggc 180

acgctggtct acaccatcgc gcagatggcg atgtaccgag gcttcaccaa gatgccccat 240

gtgcagtaca tccatacgga agcttccgag agtctctgtg gccttaagct ggaggtcaac 300

aagtaccagt acctgctgac aggtcgcgtc tatgatggca agatgtacac ggggctgtgc 360

aacttcgtgg agaggtggga ccagctcacc ctctcccagc gcaaggggct gaactatcgg 420

tatcacctgg gttgtaactg caagatcaag tcctgctact acctgccttg ctttgtgact 480

tccaagaacg agtgtctctg gaccgacatg ctctccaatt tcggttaccc tggctaccag 540

tccaaacact acgcctgcat ccggcagaag ggcggctact gcagctggta ccgaggatgg 600

gcccccccgg ataaaagcat catcaatgcc acagacccct ctagaacaac ctgtatccca 660

agcagcggtc attcaagaca cagatatgca cttataccca taccattagc agtaattaca 720

acatgtattg tgctgtatat gaatgtatta tagtcgac 758

<210> 5
<211> 1314
<212> DNA
<213> Artificial Sequence

<220>
<223> Truncated human TIMP-1 fused to fractalkine domain and GPI
      sequence

<400> 5
gaattcatgg ccccctttga gcccctggct tctggcatcc tgttgttgct gtggctgata 60

gcccccagca gggcctgcac ctgtgtccca ccccacccac agacggcctt ctgcaattcc 120

gacctcgtca tcagggccaa gttcgtgggg acaccagaag tcaaccagac caccttatac 180

cagcgttatg agatcaagat gaccaagatg tataaagggt tccaagcctt aggggatgcc 240

gctgacatcc ggttcgtcta cacccccgcc atggagagtg tctgcggata cttccacagg 300

tcccacaacc gcagcgagga gtttctcatt gctggaaaac tgcaggatgg actcttgcac 360

atcactacct gcagttttgt ggctccctgg aacagcctga gcttagctca gcgccggggc 420

ttcaccaaga cctacactgt tggctgtgag gaatccacag tgtctagagg cggcaccttc 480

gagaagcaga tcggcgaggt gaagcccagg accacccctg ccgccggggg aatggacgag 540

tctgtggtcc tggagcccga agccacaggc gaaagcagta gcctggagcc gactccttct 600

tcccaggaag cacagagggc cctggggacc tccccagagc tgccgacggg cgtgactggt 660

tcctcaggga ccaggctccc cccgacgcca aaggctcagg atggagggcc tgtgggcacg 720

gagcttttcc gagtgcctcc cgtctccact gccgccacgt ggcagagttc tgctccccac 780

caacctgggc ccagcctctg ggctgaggca aagacctctg aggccccgtc cacccaggac 840

ccctccaccc aggcctccac tgcgtcctcc ccagccccag aggagaatgc tccgtctgaa 900

ggccagcgtg tgtggggtca gggacagagc cccaggccag agaactctct ggagcgggag 960

gagatgggtc ccgtgccagc gcacacggat gccttccagg actgggggcc tggcagcatg 1020

gcccacgtct ctgtggtccc tgtctcctca gaagggaccc ccagcaggga gccagtggct 1080

tcaggcagct ggacccctaa ggctgaggaa cccatccatg ccaccatgga cccccagagg 1140

ctgggcgtcc ttatcactcc tgtccctgac gcccaggctg ccacccggag gcaggctaga 1200

acaacctgta tcccaagcag cggtcattca agacacagat atgcacttat acccatacca 1260

ttagcagtaa ttacaacatg tattgtgctg tatatgaatg tattatgagt cgac 1314